Europäisches Patentamt

European Patent Office

Office européen des brevets

(11) Publication number: **0 267 470**
**A1**

## (12) EUROPEAN PATENT APPLICATION

(21) Application number: 87115413.4

(51) Int. Cl.4: **C03C 25/00** , C12N 11/14

(22) Date of filing: 21.10.87

(30) Priority: 03.11.86 US 926156
28.01.87 US 78848
19.08.87 US 87535

(43) Date of publication of application:
18.05.88 Bulletin 88/20

(84) Designated Contracting States:
AT BE CH DE ES FR GB GR IT LI LU NL SE

(71) Applicant: **MANVILLE CORPORATION**
**Ken-Caryl Ranch 12999 Deer Creek Canyon Road**
**Denver, CO 80217(US)**

(72) Inventor: **Chelland, Joseph John**
**6655 W. Glasgow Avenue**
**Littleton Colorado 80123(US)**

(74) Representative: **Grättinger, Günter**
**Wittelsbacherstrasse 5 Postfach 16 49**
**D-8130 Starnberg(DE)**

(54) **Porous glass fiber mats for attachment of cells and biologically active substances.**

(57) A glass fiber mat comprising a plurality of glass fibers bonded together with an inorganic binder, the bonded glass fibers having been treated with an acid. Preferably, the inventive mat comprises a mixture of glass fibers having mean diameters between about 0.05 to 3.5 microns and between about 8 to 25 microns.

The inventive mat is very suitable for attachment or immobilizing of cells and biologically substances.

EP 0 267 470 A1

# POROUS GLASS FIBER MATS FOR ATTACHMENT OF CELLS AND BIOLOGICALLY ACTIVE SUBSTANCES

This is a Continuation-in-Part of Application Serial No. 07/007,848 filed January 28, 1987 which is a Continuation-in-Part of Application Serial No. 06/926,156 filed November 3, 1986, now abandoned.

## Field of the Invention

This invention relates to a porous, glass fiber mat and more particularly, it relates to a porous, glass fiber mat useful for attaching enzymes, cells and the like. It also relates to a glass fiber mat which has enzymes, cells, and the like attached thereto.

## Background of the Invention

The use of various substances to support, and in some instances immobilize, catalytically active materials is well known to those skilled in the art. Since catalytically active substances help to make reactions proceed which would otherwise not be thermodynamically possible or economically practical in many substances, it has become increasingly important to look for ways to efficiently utilize and maintain such catalytically active materials. Furthermore, since the cost of the catalytically active materials must itself be an active consideration in deciding whether to commercialize a process using the catalyst, there is even more reason to look at utilizing the catalyst as desirably as possible.

One of the most important classes of catalytically active materials or agents currently being studied and utilized are enzymes. It is known that enzymes, which are proteinaceous in nature and which are commonly water soluble, act as biocatalysts which serve to regulate many and varied chemical reactions which occur in living organisms. The enzymes may also be isolated and used in analytical, medical, and industrial applications. For example, they find use in industrial applications in the preparation of food such as cheese or bread as well as the preparation of alcoholic beverages. The enzyme glucose isomerase is extensively used to convert glucose to fructose in the manufacture of high fructose corn syrup.

Since enzymes are commonly water soluble as well as generally unstable, and therefore subject to deactivation, they are difficult to remove for reuse from solutions in which they are utilized and they may not retain their catalytic activity over extended periods of time. These difficulties lead to an increased cost in the use of enzymes in commercial scale operations due to the necessity for frequent replacement of the enzyme. In order to reduce the high cost of enzyme replacement, various methods to immobilize enzymes prior to their use have been devised. Immobilization of the enzyme permits its reuse, whereas it might otherwise undergo deactivation or be lost in the reaction medium in which it is used. Immobilized enzyme systems may be employed in various reactor systems, for example, in packed columns and stirred tank reactors, depending on the nature of the substrate which is being biochemically reacted.

Apart from immobilization of the enzymes themselves, various substances and techniques have been put forward by which the enzymes could be immobilized without isolation. In particular, whole cells of micro-organisms can be immobilized, thus using the microbial cell as a carrier for the enzyme and obviating the need for extraction of the enzyme from the cell.

One commonly used support or entrapment material for microbial cell immobilization is a gel, usually an alginate gel. Essentially the cells are trapped in a three-dimentional polymer network with relatively large interstitial spaces in the gel. The use of such gels has not been without problems though.

One problem with immobilizing microbial cells in a gel is their tendency to lose their activity during storage or other periods of non-use, for instance during transportation. An accompanying difficulty during non-use is the tendency for contaminating micro-organisms to proliferate. It is a relatively routine matter to prepare gel-immobilized cells which have high activity upon immediate use, but the activity tends to decay relatively quickly if the gel-immobilized microbial cells are not used. A basic disadvantage of gel is that it has a high water activity and probably provides a good environment for growth of contaminant moulds, bacteria, and the like. Such gels, of course, are not reusable either.

Another type of material used to immobilize catalytic agents such as enzymes and microbial cells is a porous pellet composed primarily of a high silica content or mixtures of silica and alumina. The high silica content is derived from the addition of a high purity siliceous material to the reaction mixture in the process of making the pellet. On the porous surfaces of the pellets are deposited small amounts of the catalytically active agent.

A disadvantage with the use of conventional high silica-based catalyst supports, though, is that their average pore diameter is too small for accommodating microbial cells. Their typical average pore diameter is much less than 1 micron. Typically, diameters of 1 to 25 microns are needed to accommodate the microbial cells. Of course, when it becomes difficult to immobilize an effective number of microbial cells on a typical silica-based catalyst support, the economic attractiveness of such a support in commercial processes is greatly reduced.

Fibers or webbing of inorganic and organic materials such as fiber glass, ceramics, cotton, nylon, rayon, acetate, wool, and polypropylene have been disclosed in U.S. Patent No. 4,407,954 to Clyde for use in supporting organisms. Whereas Clyde discloses the general use of such materials for supporting organisms, its disclosure also addresses the common place problem that when organisms are attached to fiber glass and other supporting materials and used in bioreactors where rather turbulent environments exist, the organisms can easily become unattached from the support and therefore are lost. Consequently, Clyde finds it necessary to move the converting organism in the bioreactor at a speed which is slightly below the speed at which the organism becomes detached from the supporting substrate.

Whereas the invention of Clyde may find applicability in the most general of applications, it has a drawback when it becomes necessary in an industrial process to move the supported organism at speeds where the organism can become detached from its support.

What is needed in the industry is an economical, easy to construct support which enzymes, cells, and the like be easily attached to and which provide suitable attachment for the enzymes and/or cells such that they do not become detached from the support in high speed and/or high shear environments common in bioreactors.

Brief Summary of the Invention

In one embodiment of the present invention, Applicant has discovered that a mat with a plurality of glass fibers bonded together with an inorganic binder wherein the bonded glass fibers have been treated with an acid provides a particularly effective support for attaching cells and biologically active substances thereto.

The inventive glass fiber mat has several advantages. To begin with, since it employs glass fibers in combination with an inorganic binder the mat is not susceptible to biological degradation as are mats composed of organic fibers and/or organic binders. Additionally, the treatment of the inorganic bonded glass fibers with an acid helps to impart some degree of rigidity to the mat such that when it is used in certain bioreactor environments it will be essentialy self-supporting and therefore will not require auxiliary supporting means. Finally, the inventive mat can be easily sterilized or disinfected because of its completely inorganic structure as opposed to mats which have organic fibers and/or binders which are susceptible to degradation when exposed to high temperatures.

In a preferred embodiment of the present invention, it has been discovered that the pore diameter and pore volume of the inventive mat can be effectively controlled by employing in the inventive mat a mixture of glass fibers, one type having a mean diameter of between about 8 and 25 microns and the other having a mean diameter of between about 0.05 to 3.5 microns. Preferably the glass fibers having a mean diameter of between about 0.05 to 3.5 microns will form from about 2 to 70 wt% of the total weight of the mixture of glass fibers.

This mixture of larger and finer diameter fibers provide an easy, effective way of controlling the pore volume and diameter of the inventive mat such that the pore volume and diameter can always be carefully controlled so as to accommodate a variety of enzymes, cells and the like of rather specific diameters. The use of the inorganic binder also helps to control pore diameter and volume because it can be conveniently utilized to fill pore spaces thereby controlling the pore diameter or volume.

Thus, Applicant's inventive mat affords a surface area suitable for attaching enzymes, cells, and the like. This custom tailoring of the pore volume and diameter to suit specific cell requirements ensures that when cells, etc., are attached to the inventive mat and used in a high speed or shear environment that the detachment of the cells from the mat will be greatly minimized.

Other aspects and advantages of the present invention are apparent from the following detailed description and claims.

Detailed Description of the Invention

Broadly, the inventive mat comprises a plurality of glass fibers bonded together with an inorganic binder, the bonded glass fibers having been treated with an acid.

Glass fibers of any size and length can be employed in the present invention. Generally, the fibers will have a mean diameter ranging from about 0.05 microns to 25 microns.

Preferably, the inventive mat comprises a mixture of two types of glass fibers. The first type comprise glass fibers of conventional form and composition. Generally, these fibers are made by a continuous filament process and are chopped to discreet and predetermined lengths for convenience in handling and easy dispersibility in forming the mat. Typically such fibers are between one quarter inch and one inch in length and have a diameter which is determined primarily by the convenience of producing these fibers in the continuous filament manner. Generally, these fibers are between 8 microns and 25 microns in mean diameter and preferably, between about 13 microns and 16 microns in mean diameter.

The other basic type of glass fiber used in the inventive mat are of much smaller size and have mean diameters ranging from 0.05 to 3.5 microns, preferably 0.6 to 1.0 microns. These glass fibers can be formed in a flame-attenuating process such as the one disclosed in U. S. Patent No. 4,167,404. After being collected, these fibers, as a mat of staple fibers, are chopped, milled or otherwise reduced in length to form a mass of glass fibers for subsequent addition to the mat. The resulting average length of these smaller fibers can be controlled to some degree. Preferably this average length should be on the order of one magnitude of the length of the larger diameter fibers used in making the mat in accordance with the present invention. However, while the larger fiber may be in the range of 1/4 to 1-1/4", preferably 1/2 to 3/4" and most preferably one half inch (1/2"), the smaller fiber lengths may range in average between one-quarter (1/4") and one-eighth inch (1/8").

The porous glass mat constructions according to the present invention are formed in a process known as the "wet" process. In the "wet" process, glass fibers of known surface characteristics, diameter, and of generally uniform length are dispersed in the form of a slurry, preferably a water slurry. The water slurry, in a known apparatus utilizing chemical dispersents and mechanical agitation, is subsequently filtered through a moving porous medium. The glass fibers thus become intertangled with one another in a jackstraw fashion as dewatered, thus forming a web. Subsequently this web, still on the moving screen, is treated with a conventional applicator device to a water suspension of an inorganic binder material, e.g. sodium or potassium silicate, and is subsequently dewatered, dried under with heated air, and then treated with an acid to form a strong, self supporting mat.

To form the mat when the two diverse types of glass fibers are used in the present invention, ordinary dispersing techniques may be employed. Conveniently, a water slurry of the larger diameter fibers (8-25 microns) is first formed and the smaller diameter fibers (0.05-3.5 microns) are added in an amount which would result in a predetermined weight percent of the resulting fibrous web being composed of the smaller fibers relatively uniformly dispersed on, between and among the larger fibers. The smaller fibers can best be dispersed by mechanically feeding a predetermined volume of the dry smaller fiber mass into the hydropulper, the subsequently metering this smaller fiber slurry into the primary water chest. Alternatively, a slurry containing one percent (1%) concentration of smaller fibers can be metered into the white water slurry itself.

With regard to the mixture of larger diameter fibers and smaller diameter fibers utilized in the present invention, preferably about 2 to 70 wt % and most preferably about 20 to 40 wt % of fibers having a mean diameter in the range of about 0.05 to 3.5 microns should be utilized based upon the total weight of the mixture of glass fibers.

The binder utilized in the present invention should be an inorganic binder. Examples of suitable inorganic binders include but are not limited to sodium silicate, potassium silicate, ethyl silicate, colloidal silica, collodial alumina, and magnesium oxide slurry set with phosphoric acid. Sodium or potassium silicate binders are preferred.

One the fibrous mat has been constructed and dried, it should be treated or saturated with an acid, either organic or inorganic. This acid treatment will help to further "set" the binder and therefore impart a degree of rigidity or self-supportiveness to the mat. The preferred inorganic acids are nitric, phosphoric, and sulfuric and the preferred organic acids are acetic acid, citric, and tartaric.

Depending upon the particular type of enzyme, cell, etc. one desires to attach to the mat, one skilled in the art can predetermine a suitable mean pore diameter and pore volume of the inventive mat by varying the type glass fiber utilized and also the amout of inorganic binder utilized in the present invention.

Generally the inventive mat will have a mean pore diameter in the range of about 20 to 200 microns, preferably about 20-100 microns, and most preferably about 25-50 microns.

The total pore volume of the inventive mat will generally be at least about 1.0 cc/g and preferably between about 1.0 cc/g to 1.6 cc/g.

The inventive mat can be formed into a wide variety of shapes which include but are not limited to sheets, plates, discs, spirals, helixes, cones, tubes, and corrugated shapes.

A variety of cells and biologically active substances can be attached and/or immobilized in the pores of the inventive mat glass fibrous material.

The term "biologically active substance" as used herein embraces proteinaceous substances (e.g. enzymes) and includes substances which are biologically active per se and those which are not, but which can be activated after immobilization to make them biologically active. In the latter case a biologically active substance can be treated with an inhibitor to deactivate it and then it can be reactivated after immobilization.

As used herein, the term "proteinaceous substances" signifies proteins per se and substances having a proteinaceous component (e.g. glyco-proteins such as amyloglucosidase and lypo-proteins.)

Furthermore, it is to be understood that the term "biologically active substance" includes inter alia those substances capable of participating in specific interactions, such substances including, for example, substances of biological origin and those which act on living organisms. Substances of synthetic origin which can participate in reactions involving specific interactions analogous to those which can occur with naturally occurring substances are also embraced within the term "biologically active substance".

Cells can also be attached/immobilized within the pores of the inventive mat as well. As used herein, the term cell includes but is not limited to microbial, mammalian, and plant cells.

Non-limiting examples of cells which can be attached/immobilized to the inventive biocarriers include bacteria, yeast, fungi, algae, pancreatic cells, and lymphocytes.

The biologically active substances and cells can be attached or immobilized within the pores of the inventive mat by any method known to those skilled in the art. For example, one may use known coupling or cross-linking agents. Alternatively, one may sometimes immobilize cells by simply contacting the mat with an aqueous suspension of cells to be immobilized thereon as there can be a natural attraction between the cell walls and the mat as produced.

Reasonable modifications or variations are possible from the foregoing disclosure without departing from either the spirit or scope of the present invention ad defined by the claims.

## Claims

1. A glass fiber mat suitable for attaching cells and biologically active substances thereto comprising a plurality of glass fibers bonded together with an inorganic binder, the bonded glass fibers having been treated with an acid.

2. A glass fiber mat according to claim 1 comprising a mixture of glass fibers have a mean diameter between about 8 and 25 microns and glass fibers have a mean diameter between about 0.05 and 3.5 microns such that said 0.05 to 3.5 mean diameter fibers are present in the fibrous mixture in an amount of from about 2 to 70 wt% of the total weight of said fibrous mixture.

3. A glass fiber mat according to claim 1 wherein said inorganic binder is selected from the group consisting of sodium and potassium silicate.

4. A glass fiber mat according to claim 1 wherein said acid is inorganic.

5. A glass fiber mat according to claim 1 having a mean pore diameter in the range of about 20 to 200 microns.

6. A glass fiber mat according to claim 5 having a mean pore diameter in the range of about 25 to 50 microns.

7. A glass fiber mat according to claim 1 having a total pore volume of at least about 1.0 cc/g.

8. A glass fiber mat according to claim 1 in the form of a sheet, disc, plate, helix, cone, tube, or corrugated shape.

European Patent Office

EUROPEAN SEARCH REPORT

Application Number

EP 87 11 5413

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 188 811 (PPG INDUSTRIES) <br> * Claim 1; page 14, lines 19-25 * <br> --- | 1,3-6,8 | C 03 C 25/00 <br> C 12 N 11/14 |
| Y | WO-A-8 604 088 (E.EHRNFORD) <br> * Claims; page 4, lines 15-29; page 5, lines 14-30 * <br> --- | 1,4-6,8 | |
| Y | US-A-3 454 453 (J.M. WARD) <br> * Example 1 * <br> ----- | 1,3-4 | |

TECHNICAL FIELDS SEARCHED (Int. Cl.4)

C 03 C 25/00
C 12 N 11/00

The present search report has been drawn up for all claims

| Place of search | Date of completion of the search | Examiner |
|---|---|---|
| THE HAGUE | 29-01-1988 | BOUTRUCHE J.P.E. |

EPO FORM 1503 03.82 (P0401)